# EUROPÄISCHE PATENTSCHRIFT

(11) **EP 0 583 726 B1**
(45) Veröffentlichungstag und Bekanntmachung des Hinweises auf die Patenterteilung: **20.11.1996**
(21) Anmeldenummer: 93112848.2
(22) Anmeldetag: 11.08.1993
(51) Int. Cl.: A61K 9/16, A61K 38/46, A61K 9/50, A61J 3/07

(54) **Pankreatinmikropellets mit magensaftresitentem Überzug**
Enteric coated pancreative micropellets
Micropilules de pancréatine avec enrobage entérique

(30) Priorität: 19.08.1992 DE 4227385
(43) Veröffentlichungstag der Anmeldung: 23.02.1994
(73) Patentinhaber: Kali-Chemie Pharma GmbH, D-30173 Hannover (DE)
(72) Erfinder: Bödecker, Bernd, D-3000 Hannover 91 (DE); Henniges, Friederike, D-3300 Braunschweig (DE); Kölln, Claus-Jürgen, D-3057 Neustadt a. Rbge (DE); Kuhnow, Günther, D-3057 Neustadt a. Rbge. (DE); Peschke, Günter-Josef, D-3000 Hannover 72 (DE); Rehburg, Manfred, D-2841 Wagenfeld 2 (DE); Sobe, Alwin, D-3203 Sarstedt (DE); Stemmle, Berthold, D-3167 Burgdorf (DE)
(74) Vertreter: Lauer, Dieter, Dr.

(56) Entgegenhaltungen:
- EP-A- 0 021 129
- EP-A- 0 141 607
- FR-A- 2 313 916
- DATABASE WPI Section Ch, Week 9233, Derwent Publications Ltd., London, GB; Class A12, AN 92-273642 & JP-A-4 187 085 (SHIONOGI & CO LTD) 3. Juli 1992

## Beschreibung

Die vorliegende Erfindung betrifft pankreatinhaltige mit einem magensaftresistenten Film überziehbare Mikropellets sowie deren Herstellung und derartige Pankreatinmikropellets enthaltende Arzneiformen.

Pankreatin ist ein Enzymgemisch mit amylolytischer, lipolytischer sowie proteolytischer Aktivität. Pankreatin kann als Arzneimittelwirkstoff zur Behandlung von Verdauungsstörungen bei Pankreas-Insuffizienz eingesetzt werden. Da Pankreatin gegenüber den Magensäften empfindlich ist, wird es vorzugsweise mit einem magensaftresistenten Film überzogen verabreicht, beispielsweise in Form von Kapseln, welche magensaftresistent überzogene Pankreatinpellets enthalten. Es ist wünschenswert, daß derartige magensaftresistent überzogene pankreatinhaltige Partikel möglichst klein sind, um einerseits eine gute Verteilung der Partikel in dem Speisebrei und eine ungehinderte Passage der Partikel durch den Pylorus zu gewährleisten und andererseits eine möglichst hohe Schüttdichte der Pankreatinpellets zu erreichen, um in den Kapseln eine möglichst große Menge Pankreatinpellets pro Kapselvolumen unterbringen zu können.

In dem Deutschen Patent Nr. 29 23 279 wird ein Verfahren zur Herstellung von Pankreatinpellets beschrieben, in welchem eine formbare Masse aus Pankreatin und organischen Lösungsmitteln auf einer Strangpresse extrudiert wird und das Extrudat in kantige Strangschnittlinge zerschnitten wird, welche durch Aufbaugranulation unter Zusatz von Pankreatinpulver abgerundet werden können.

Es wurde nun ein verbessertes Verfahren gefunden, mit welchem neue magensaftresistente filmüberzogene Pankreatinmikropellets mit einer hohen Schüttdichte und einer eine gute Passage durch den Pylorus gewährleistenden kleinen Partikelgröße hergestellt werden können.

Die vorliegende Erfindung betrifft daher neue mit einem magensaftresistenten Film überziehbare Pankreatinmikropelletkerne mit einem Pankreatingehalt von 65-85, insbesondere 75-80 Gew.-%, dadurch gekennzeichnet, daß sie eine Schüttdichte von 0,6 g/ml bis 0,85 g/ml besitzen und daß sie im wesentlichen aus Pankreatin, Polyethylenglykol 4000 und dünnflüssigem Paraffin bestehen und auf 100 Gew.-Teile Pankreatin 15-50, inbesondere 20-30 Gew.-Teile Polyethylenglycol 4000 und 1,5-5, insbesondere 2-3 Gew.-Teile dünnflüssiges Paraffin enthalten und daß sie eine kugelige bis ellipsoide Gestalt haben, wobei der Kugeldurchmesser bzw. die kurze Achse im Bereich von 0,7-1,4 mm, insbesondere 0,8-1,2 mm liegt, und eine Partikelgrößenverteilung haben, bei der mindestens 80 % der Pankreatinmikropelletkerne ein Verhältnis von kurzer Achse zur langen Achse im Bereich von 1:1 bis 1:2 aufweisen.

Ferner betrifft die Erfindung magensaftsresistente filmüberzogene Pankreatinpellets, welche aus den vorgenannten Pankreatinmikropelletkernen und einem magensaftresistenten Filmüberzug bestehen.

Weiterhin betrifft die Erfindung ein Verfahren zur Herstellung der vorstehend beschriebenen mit einem magensaftresistenten Film überziehbaren Pankreatinmikropelletkernen, dadurch gekennzeichnet, daß man
a) 100 Gew.-Teile Pankreatin mit 15-50, insbesondere 20-30 Gew.-Teilen Polyethylenglykol 4000 und einer zur Erzielung einer extrudierbaren Konsistenz ausreichenden Menge eines niederen Alkohols, insbesondere Propan-2-ol, zu einer extrudierbaren Mischung vermischt
b) die extrudierbare Mischung in einer Strangpresse mit einer Lochmatrize mit einem zur Erreichung des vorstehend angegebenen Pelletkerndurchmesserbereiches geeigneten Lochdurchmesser, insbesondere einem Lochdurchmesser von 0,8-1,2 mm zu Strängen verpresst und der Presse Strangbruchstücke einer zur Überführung in ein Rundungsgerät geeigneten Länge entnimmt und
c) die Strangbruchstücke in ein Rundungsgerät überführt und darin unter Hinzufügung von 1,5-5, insbesondere 2-3 Gew.-Teilen dünnflüssigem Paraffin und 1,5-10, insbesondere 2-6 Gew.-Teilen Propan-2-ol, jeweils bezogen auf 100 Gew.-Teile Pankreatin, unter Bruchkanten rundenden Bedingungen zu Mikropelletkernen von kugeliger bis ellipsoider Gestalt mit einer Partikelgrößenverteilung, bei der mindestens 80 % der Partikel ein Verhältnis von kurzer Achse zur langen Achse im Bereich von 1:1 bis 1:2 aufweisen, zerbricht und
d) die unter c) erhaltenen Mikropelletkerne bei Temperaturen im Bereich von 30-50 °C trocknet.

Nach dem erfindungsgemäßen Verfahren werden Pankreatinmikropelletkerne, welche zur Herstellung von magensaftresistenten filmüberzogenen Pankreatinmikropellets anschließend auf an sich bekannte Weise mit einem magensaftresistenten Film überzogen werden können, erhalten. Nach dem erfindungsgemäßen Verfahren wird das Pankreatin zunächst mit dem Polyethylenglykol sowie einem niederen Alkohol, vorzugsweise Propan-2-ol, zu einer extrudierbaren Mischung vermischt. Vorzugsweise werden auf 100 Gew.-Teile Pankreatin 25 (± 20 %) Gew.-Teile Polyethylenglykol 4000 eingesetzt. Das als Granulierflüssigkeit dienende Propan-2-ol, welches anschließend wieder verdunstet und/oder durch Trocknung entfernt wird, wird in solcher Menge zugesetzt, daß das Gemisch ausreichend angefeuchtet wird, um eine extrudierbare Masse zu ergeben. Im allgemeinen wird dies mit 10 - 30 Gew.-Teilen, vorzugsweise 15-25 Gewichtsteilen, Propan-2-ol bezogen auf 100 Gew.-Teile Pankreatin, erreicht. Gewünschtenfalls können der extrudierbaren Mischung auch weitere bekannte pharmazeutisch übliche Hilfsstoffe wie z.B. übliche Konservierungsmittel wie Parahydroxybenzoesäureester zugesetzt werden.

Die extrudierbare Mischung wird anschließend in einer an sich bekannten Strangpresse, welche mit einer Lochmatrize mit einem Lochdurchmesser im Bereich zwischen 0,8 und 1,2 mm versehen ist, auf an sich bekannte Weise extrudiert. Um zu vermeiden, daß die auftretende Kompressionswärme die Enzyme schädigt, kann die Kompressionswärme durch zusätzliche Kühlvorrichtungen an der Strangpresse abgeführt werden, und das zu extrudierende Gemisch kann gewünschtenfalls vorgekühlt werden. Der Maschinendurchsatz ist von der Größe des Extruders und der Pankreatinqualität abhängig. Übliche Maschinendurchsätze können so beispielsweise im Bereich von 15-150 kg/h liegen.

Die Querschnittsfestigkeit der aus der Lochmatrize austretenden Extrudatstränge ist gering genug, daß die Stränge noch in der Strangpresse von selbst in Strangbruchstücke mit einer für die Weiterführung in ein Rundungsgerät geeigneten Länge zerbrechen können. Andererseits ist die Querschnittfestigkeit der Extrudatstränge hoch genug, daß kein zu starkes Zerbröseln unter Bildung eines unerwünscht hohen Feinanteils stattfindet. Gewünschtenfalls kann auch eine Steuerung der Bruchstücklänge dadurch erzielt werden, daß in der Strangpresse hinter der Lochmatrize eine Brech- oder Schneidvorrichtung für die Stränge angebracht wird.

Die Querschnittfestigkeit der Strangbruchstücke ist derart, daß sie bei der Weiterbehandlung in einem Rundungsgerät, beispielsweise einem handelsüblichen Spheronizer, zu Partikeln der gewünschten Pelletgröße weiter zerbrechen. Die Länge der Strangbruchstücke ist für die Weiterverarbeitung in dem Rundungsgerät dabei unwesentlich. Um jedoch einen ungehinderten Zufluß der Strangbruchstücke in das Rundungsgerät zu gewährleisten, ist es zweckmäßig, wenn die Länge zumindest des Hauptanteiles der Strangbruchstücke 5 cm nicht überschreitet und vorzugsweise im Bereich zwischen 0,5 und 3 cm liegt, um ein stärkeres Verhaken der Strangbruchstücke untereinander und damit eine Störung der gleichmäßigen Überführung des Gutes in das Rundungsgerät zu vermeiden.

Die Behandlung der Strangbruchstücke in dem Rundungsgerät erfolgt auf an sich bekannte Weise. Hierbei werden die Strangbruchstücke in kleine Partikel weiter zerbrochen und diese gerundet, wobei man zu den Strangbruckstücken im Rundungsgerät gleichzeitig 1,5-5, insbesondere 2-3 Gew.-Teile dünnflüssiges Paraffin und eine zum Ausgleich der Verdunstung während der Verweilzeit im Rundungsgerät ausreichende Menge an Propan-2-ol im Bereich von 1,5-10, insbesondere 2-6 Gew.-Teilen, jeweils bezogen auf 100 Gew.-Teile Pankreatin, zufügt.

Die Querschnittsfestigkeit der Strangbruchstücke ist erstaunlicherweise derart, daß bei dem weiteren Zerbrechen in dem Rundungsgerät Partikel von kugeliger bis ellipsoider Gestalt mit einem sehr engen Partikelgrößenspektrum entstehen, bei dem mindestens 80 % der gebildeten Partikel ein Verhältnis von kurzer Achse zur langen Achse von 1:1 bis 1:2 aufweisen. Auch die Ausrundung der Partikel ist zufriedenstellend.

Nach der Ausrundung werden die noch feuchten Rundpellets bei 30-50 °C, insbesondere bei 40 °C, in einer üblichen Trocknungsanlage, beispielsweise einem Hordenblechtrockner, getrocknet, um Propan-2-ol aus den Rundpellets weitgehend zu entfernen. Üblicherweise wird getrocknet, bis der Gehalt an Propan-2-ol in den Rundpellets kleiner gleich 1 % ist. Durch das erfindungsgemäße Verfahren werden so auf problemlose Weise mit einem magensaftresistenten Film überziehbare Pankreatinmikropelletkerne erhalten.

Die erfindungsgemäße Kombination der Zusammensetzung der zu extrudierenden Mischung und des geringen Querschnitts der Extrudatstränge führt dazu, daß das beim Extrudieren auf der Strangpresse erhaltene Extrudat in Form von Strängen anfällt, deren Querschnittsfestigkeit genauso eingestellt ist, daß die Stränge eine ausreichende Teilungsbereitschaft besitzen, um in der Strangpresse in Strangbruchstücke mit einer zur Überführung in ein Rundungsgerät geeigneten Länge zerbrechen zu können und die Strangbruchstücke bei der Behandlung in einem Rundungsgerät weiter zu Partikeln zerbrechen, welche das vorstehend angegebene enge Partikelgrößenspektrum aufweisen und eine solche Konsistenz besitzen, daß sie bei der Behandlung in dem Rundungsgerät ausreichend gerundet werden können, so daß bei den gebildeten Pellets keine scharfen Kanten oder Höhlen bestehen bleiben, und sie anschließend problemlos mit einem Film überzogen werden können. Es ist überraschend, daß erfindungsgemäß ein Extrudat hergestellt wird, welches genau den Grad an Brüchigkeit aufweist, welcher bei der Weiterverarbeitung zu Mikropelletkernen der gewünschten Größe, welche gute Ausrundungseigenschaften besitzen, führt, ohne daß bei einer Strangpress-Agglomeration durch Lochmatrizen mit so geringem Durchmesser, bei welcher hohe Kompressionsdrücke auftreten, eine Schädigung der Enzym-Aktivität des Pankreatin auftritt, oder das Extrudat derart verhärtet wird, daß bei der anschließenden Behandlung in dem Rundungsgerät das Weiterzerbrechen der Strangbruchstücke bishin zu den gewünschten Mikropelletkerngrößen und die Abrundung der Bruchkanten wegen einer zu großen Härte des Materials behindert wäre und keine zufriedenstellende Formgebung mehr gelingen würde.

Das erfindungsgemäße Verfahren bietet somit den Vorteil, daß beim Strangpressen Strang-Agglomerate erhalten werden, welche ohne einen zusätzlichen Schneidvorgang zu Strangbruchstücken von zur Weiterverarbeitung geeigneter Länge zerbrechen können und diese sich ohne weiteren Arbeitsgang bei der anschließenden Behandlung in einem Rundungsgerät in Partikel der gewünschten Mikropelletgröße mit einem überraschend engen Partikelgrößenspektrum und guten Ausrundungseigenschaften weiterbrechen lassen. Dabei wird durch die Vermeidung von Feinanteilanfall und die Erzielung eines engen Partikelgrößenspektrums eine hohe Ausbeute an Mikropelletkernen erzielt. Trotz der geringen Lochdurchmesser der Lochmatrize kann eine hohe Pelletierleistung erreicht werden.

Die erfindungsgemäß erhaltenen Pankreatinmikropelletkerne können auf an sich bekannte Weise mit einem magensaftresistenten Überzug versehen werden. Beispielsweise können die Pankreatinmikropelletkerne mit an sich bekannten magensaftresistenten Filmbildnern wie z. B. Hydroxypropylmethylcelluloseacetatsuccinat (= HPMCAS), Hydroxypropylmethylcellulosephthalat (=HPMCP), Celluloseacetatphthalat (=CAP) oder Polyvinylacetatphthalat (=PVAP) überzogen werden. Auch die an sich bekannten als Filmbildner üblichen Copolymere wie beispielsweise Methacrylsäure-Methylmethacrylat-Copolymerisate oder Methacrylsäure-Ethylacrylat-Copolymerisate sind möglich. Die Filmbildner können dabei in den üblichen Anwendungsformen, z. B. als organische Lösungen oder organische oder wäßrige Dispersionen, gegebenenfalls unter Zusatz eines gebräuchlichen Weichmachers, auf die erfindungsgemäßen Pankreatinmikropelletkerne in an sich bekannten Befilmungsapperaturen, z. B. Kugelcoatern, aufgebracht werden.

Die erhaltenen magensaftresistenten filmüberzogenen Pankreatinmikropellets zeichnen sich durch eine hohe Schüttdichte, beispielsweise im Bereich von 0,6 g/ml bis 0,85 g/ml aus, dies ermöglicht es, das Füllgewicht pro Kapsel und damit deren Wirkstoffgehalt zu erhöhen.

Das nachfolgende Beispiel soll die Erfindung näher erläutern, ohne deren Umfang zu beschränken.

### Beispiel

120 kg Pankreatin wurden in einem handelsüblichen Mischer mit 30 kg Polyethylenglykol 4000 gemischt und mit ca. 20 kg Propan-2-ol durchfeuchtet.

Die Mischung wurde durch eine Strangpresse (Extruder), welche mit einer Lochmatritze mit Bohrungen von 0,8 mm lichter Weite und einer dahinterliegenden Schneidevorrichtung ausgestattet war, gedrückt. Hierbei wurden Strangbruchstücke mit einer Stranglänge von bis zu 20 mm erhalten.

In Portionen von je ca. 15 kg wurden die Strangbruchstücke in einem Rundungsgerät (Typ Caleva) zerbrochen und zu sphärisch geformten Pellets ausgerundet, wobei man jeder Portion noch 300 g dünnflüssiges Paraffin und abhängig von der Verweilzeit im Rundungsgerät (3-6 Min.) noch ca. 300 bis 700 g Propan-2-ol zufügte.

Nach der Trocknung in einem handelsüblichen Hordenblechtrockner erhielt man als Ausbeute ca. 90 % an Pankreatinmikropelletkernen mit einem Durchmesser von 0,7 bis 1,4 mm, klassiert mit einem 0,7 mm-Sieb (Absiebung von Unterkorn < 0,7 mm) und einem 1,4 mm-Sieb (Absiebung von Überkorn > 1,4 mm), mit einem Pankreatingehalt von ca. 78 %. Die Schüttdichte betrug 0,7 g/ml.

Anschließend wurden die Mikropelletkerne in einer üblichen Befilmungsapparatur mit einer Lösung aus Hydroxypropylmethylcellulosephtalat (Typ HP55), Dibutylphtalat, dünnflüssigem Paraffin und Silikonöl (Dimethicone 1000) in Aceton auf an sich bekannte Weise magensaftresistent überzogen. Als Ausbeute erhielt man ca. 90 % magensaftresistente Pankreatinmikropellets, mit einem Durchmesser im Bereich von 0,7 bis 1,6 mm, klassiert mit einem 0,7 mm-Sieb (Absiebung von Unterkorn < 0,7 mm) und einem 1,6 mm-Sieb (Absiebung von Überkorn > 1,6 mm) mit einem Gehalt von ca. 60 % Pankreatin, bezogen auf die filmüberzogenen Mikropellets, und einer Schüttdichte von 0,8 g/ml.

## Patentansprüche

1. Mit einem magensaftresistenten Film überziehbare Pankreatinmikropelletkerne mit einem Pankreatingehalt von 65-85, insbesondere 75-80 Gew.%, dadurch gekennzeichnet, daß sie eine Schüttdichte von 0,6 g/ml bis 0,85 g/ml besitzen und daß sie im wesentlichen aus Pankreatin, Polyethylenglykol 4000 und dünnflüssigem Paraffin bestehen und auf 100 Gew.-Teile Pankreatin 15-50, insbesondere 20-30 Gew.-Teile Polyethylenglykol 4000 und 1,5-5, insbesondere 2-3 Gew.-Teile dünnflüssiges Paraffin enthalten und daß sie eine kugelige bis ellipsoide Gestalt haben, wobei der Kugeldurchmesser bzw. die kurze Achse im Bereich von 0,7-1,4 mm, insbesondere 0,8-1,2 mm liegt, und eine Partikelgrößenverteilung haben, bei der mindestens 80 % der Pankreatinmikropelletkerne ein Verhältnis von kurzer Achse zur langen Achse im Bereich von 1:1 bis 1:2 aufweisen.

2. Magensaftresistente filmüberzogene Pankreatinpellets, dadurch gekennzeichnet, daß sie aus Pankreatinmikropelletkernen gemäß Anspruch 1 und einem magensaftresistenten Filmüberzug bestehen.

3. Verfahren zur Herstellung von mit einem magensaftresistenten Film überziehbaren Pankreatinmikropelletkernen mit einem Pankreatingehalt von 65-85, insbesondere 75-80 Gew.-%, dadurch gekennzeichnet, daß man
a) 100 Gew.-Teile Pankreatin mit 15-50, insbesondere 20-30 Gew.-Teilen Polyethylenglykol 4000 und einer zur Erzielung einer extrudierbaren Konsistenz ausreichenden Menge eines niederen Alkohols, insbesondere Propan-2-ol, zu einer extrudierbaren Mischung vermischt
b) die extrudierbare Mischung in einer Strangpresse mit einer Lochmatrize mit Lochdurchmesser von 0,8-1,2 mm zu Strängen verpresst und der Presse Strangbruchstücke einer zur Überführung in ein Rundungsgerät geeigneten Länge entnimmt und
c) die Strangbruchstücke in ein Rundungsgerät überführt und darin unter Hinzufügung von 1,5-5, insbesondere 2-3 Gew.-Teilen dünnflüssigem Paraffin und 1,5-10, insbesondere 2-6 Gew.-Teilen Propan-2-ol, jeweils bezogen auf 100 Gew.-Teile Pankreatin, unter Bruchkanten rundenden Bedingungen zu Mikropelletkernen von kugeliger bis ellipsoider Gestalt mit einer Partikelgrößenverteilung, bei der mindestens 80 % der Partikel ein Verhältnis von kurzer Achse zur langen Achse im Bereich von 1:1 bis 1:2 aufweisen, zerbricht und
d) die unter c) erhaltenen Mikropelletkerne bei Temperaturen im Bereich von 30-50 °C trocknet.

4. Verfahren nach Anspruch 3, dadurch gekennzeichnet, daß die der Strangpresse entnommenen Strangbruchstücke eine Länge von höchstens 5 cm, vorzugsweise im Bereich von 0,5-3 cm besitzen.

5. Verfahren nach Anspruch 3, dadurch gekennzeichnet, daß in der Strangpresse die aus der Lochmatrize austretenden Stränge durch ein hinter der Lochmatrize befindliches Schneidgerät zerteilt werden.

6. Verfahren nach Anspruch 3, dadurch gekennzeichnet, daß in Verfahrensschritt a) Propan-2-ol in einer Menge von 10 bis 30 Gew.-Teilen, vorzugsweise 15-25 Gew.-Teilen, bezogen auf 100 Gew.-Teile Pankreatin zugemischt werden.

7. Verfahren nach Anspruch 3, dadurch gekenzeichnet, daß die im Verfahrensschritt c) erhaltenen Pankreatinmikropelletkerne mit einem magensaftresistenten Film überzogen werden.

## Claims

1. Pancreatin micropellet cores which can be coated with a gastric juice-resistant film and having a pancreatin content of 65-85, in particular 75-80, % by weight, characterised in that they have a bulk density of 0.6 g/ml to 0.85 g/ml and that they consist essentially of pancreatin, polyethylene glycol 4000 and highly liquid paraffin and contain, relative to 100 parts by weight of pancreatin, 15-50, in particular 20-30, parts by weight of polyethylene glycol 4000 and 1.5-5, in particular 2-3, parts by weight of highly liquid paraffin and in that they have a spherical to ellipsoidal shape, the sphere diameter or the minor axis being in the range from 0.7-1.4 mm, in particular 0.8-1.2 mm, and have a particle size distribution in which at least 80% of the pancreatin micropellet cores have a ratio of the minor axis to the major axis in the range 1:1 to 1:2.

2. Gastric juice-resistant film-coated pancreatin pellets, characterised in that they consist of pancreatin micropellet cores according to Claim 1 and a gastric juice-resistant film coating.

3. Process for the production of pancreatin micropellet cores which can be coated with a gastric juice-resistant film and having a pancreatin content of 65-85, in particular 75-80, % by weight, characterised in that
a) 100 parts by weight of pancreatin are mixed with 15-50, in particular 20-30, parts by weight of polyethylene glycol 4000 and an amount of a lower alcohol, in particular propan-2-ol, adequate to achieve an extrudable consistency, to give an extrudable mixture,
b) the extrudable mixture is pressed in an extruding press containing a piercing die having a hole diameter of 0.8-1.2 mm to give extrudates, and extrudate fragments of a length suitable for transfer to a rounding apparatus are taken from the press and
c) the extrudate fragments are transferred to a rounding apparatus and broken up therein with the addition of 1.5-5, in particular 2-3, parts by weight of highly liquid paraffin and 1.5-10, in particular 2-6, parts by weight of propan-2-ol, in each case relative to 100 parts by weight of pancreatin, under conditions which round fracture edges, to give micropellet cores of spherical to ellipsoidal shape having a particle size distribution in which at least 80% of the particles have a ratio of the minor axis to the major axis in the range 1:1 to 1:2, and
d) the micropellet cores obtained in c) are dried at temperatures in the range 30-50°C.

4. Process according to Claim 3, characterised in that the extrudate fragments taken from the extruding press have a length of at most 5 cm, preferably in the range 0.5-3 cm.

5. Process according to Claim 3, characterised in that in the extruding press the extrudates emerging from the piercing die are divided by a cutting apparatus located after the piercing die.

6. Process according to Claim 3, characterised in that in process step a) propan-2-ol is admixed in an amount from 10 to 30 parts by weight, preferably 15-25 parts by weight, relative to 100 parts by weight of pancreatin.

7. Process according to Claim 3, characterised in that the pancreatin micropellet cores obtained in process step c) are coated with a gastric juice-resistant film.

## Revendications

1. Noyaux de micropillules de pancréatine pour enrobage par un film résistant aux sucs gastriques avec une teneur en pancréatine de 65 à 85, en particulier 75 à 80 % en poids, caractérisés en ce qu'ils possèdent une masse volumique apparente de 0,6 à 0,85 g/ml, en ce qu'ils sont constitués en substance de pancréatine, de polyéthylène glycol 4000 et de paraffine fluide et contiennent, sur 100 parties en poids de pancréatine, 15 à 50, en particulier 20 à 30 parties en poids de polyéthylène glycol 4000 et 1,5 à 5, en particulier 2 à 3 parties en poids de paraffine fluide, et en ce qu'ils ont une forme sphérique ou ellipsoïdale, le diamètre des sphères ou l'axe court se situant dans la plage de 0,7 à 1,4 mm, en particulier de 0,8 à 1,2 mm, et ont une distribution de calibres particulaires dans laquelle au moins 80 % des noyaux de micropillules de pancréatine présentent un rapport de l'axe court à l'axe long dans la plage de 1:1 à 1:2.

2. Micropillules de pancréatine enrobées d'un film résistant aux sucs gastriques, caractérisées en ce qu'elles sont constituées de noyaux de micropillules de pancréatine selon la revendication 1 et d'un enrobage par un film résistant aux sucs gastriques.

3. Procédé de préparation de noyaux de micropillules de pancréatine pour enrobage par un film résistant aux sucs gastriques avec une teneur en pancréatine de 65 à 85, en particulier de 75 à 80 % en poids, caractérisé par les étapes suivantes :
a) on mélange 100 parties en poids de pancréatine avec 15 à 50, en particulier 20 à 30 parties en poids de polyéthylène glycol 4000 et avec une quantité, suffisante pour obtenir une consistance extrudable, d'un alcool inférieur, en particulier du propane-2-ol, pour former un mélange extrudable,
b) on presse le mélange extrudable dans une boudineuse équipée d'une matrice perforée avec un diamètre des trous de 0,8 à 1,2 mm pour former des boudins et on retire de la boudineuse des fragments de boudins d'une longueur convenant à un transfert dans un appareil d'arrondissage, et
c) on transfère les fragments de boudins dans un appareil d'arrondissage où, après addition de 1,5 à 5, en particulier 2 à 3 parties en poids de paraffine fluide et de 1,5 à 10, en particulier 2 à 6 parties en poids de propane-2-ol, respectivement par rapport à 100 parties en poids de pancréatine, dans des conditions d'arrondissage des arêtes des fragments, ils sont fractionnés en noyaux de micropillules de forme sphérique à ellipsoïdale avec une distribution des calibres particulaires dans laquelle au moins 80 % des particules présentent un rapport de l'axe court à l'axe long dans la plage de 1:1 à 1:2, et
d) on sèche les noyaux de micropillules obtenus sous c) à des températures de 30 à 50°C.

4. Procédé selon la revendication 3, caractérisé en ce que les fragments de boudins retirés de la boudineuse ont une longueur au maximum de 5 cm, de préférence dans la plage de 0,5 à 3 cm.

5. Procédé selon la revendication 3, caractérisé en ce que les boudins sortant de la matrice perforée, dans la boudineuse, sont fractionnés par un appareil de coupe se trouvant derrière la matrice perforée.

6. Procédé selon la revendication 3, caractérisé en ce que, dans l'étape a) du procédé, on ajoute du propane-2-ol en quantité de 10 à 30 parties en poids, de préférence 15 à 25 parties en poids par rapport à 100 parties en poids de pancréatine.

7. Procédé selon la revendication 3, caractérisé en ce que les noyaux de micropillules de pancréatine obtenus dans l'étape c) du procédé sont enrobés d'un film résistant aux sucs gastriques.
